# EUROPEAN PATENT APPLICATION

(11) **EP 2 604 123 A1**
(43) Date of publication of application: **19.06.2013**
(21) Application number: 11193842.9
(22) Date of filing: 15.12.2011
(51) Int. Cl.: A23C 9/123, A23L 1/30, A23L 1/304, A61P 1/12, A61K 33/06, A61K 35/74

(54) **Method and nutritional compositions for the treatment of diarrhea.**

(71) Applicant: Friesland Brands B.V., 3818 LE Amersfoort (NL)
(72) Inventor: van den Heuvel, Elisabeth Gertruda Hendrika Maria, 3818 LE Amersfoort (NL); Agustina, Rina, 10430 Jakarta Pusat (ID); Kok, Franciscus Johannes, 6866 GD Heelsum (NL); Bovee-Oudenhoven, Ingeborg Marie Jacqueline, 6718 ZB Ede (NL); Albers, Ruud, 3235 KT Rockanje (NL)
(74) Representative: Jansen, Cornelis Marinus

(57) **Abstract**

The invention relates to methods for reducing the duration of an episode of diarrhea in an individual comprising administering to said individual viable *Lactobacillus casei* CRL 431 in an amount of at least 10⁶ CFU per 24 hours and calcium in an amount of at least 300 mg per 24 hours. The invention further relates to nutritional compositions comprising viable *Lactobacillus casei* CRL 431 in an amount of at least 10⁴ CFU per ml and calcium in an amount of at least 0.6 mg per ml which is suitable for the treatment of diarrhea. The composition may be in the form of a kit of parts.

## Description

The invention relates to methods for treating diarrhea and to nutritional compositions, such as liquid nutritional compositions, useful in treating diarrhea. More specifically, it relates to reducing the duration of diarrheal episodes.

Diarrhea is a serious health problem worldwide. Each year, there are about 4 billion cases of diarrhea in children and adults and about 2.2 million deaths occur worldwide due to diarrhea. Diarrhea related morbidity and mortality mostly occur in the developing world. However, also in the developed world many experience one or more episodes of diarrhea, for instance about 272 million individuals in the US are affected by diarrhea annually (Shastri *et al*. 2008).

Acute diarrhea is characterized by a suddenly occurring deviation from the defecation pattern typical for a person with a maximum duration of 14 days, while the frequency and the amount of feces are increased and the feces contain more water than usual. Chronic diarrhea has the symptoms of acute diarrhea, but generally lasts longer than 14 days. Acute diarrhea may be caused by a variety of factors, including viral or bacterial infections, allergic reaction, reaction to medication, such as antibiotics. Causes of chronic include intestinal diseases, such as ulcerative colitis, Crohn's disease, irritable bowel syndrome, inflammatory bowel disease and celiac disease.

During diarrhea, normal intestinal physiology is disturbed due to the presence and colonization of pathogens resulting in altered intestinal water absorption and secretion, mucosal barrier permeability, mucosal host defence mechanisms and intestinal inflammation.

Rotavirus infection is the most important cause of diarrheal illness in small children and of severe diarrhea-related hospitalization among children in both developed and developing countries. In addition to rotavirus, several other viruses are known to cause diarrhea, such as noroviruses (e.g. Norwalk virus), enteric adenoviruses and astroviruses. Bacterial infections are an important cause of acute diarrhea in developing countries as well and may also lead to severe disease. Among the main bacterial agents of diarrheal diseases are *Vibrio cholerae* (cholera), a variety of *Salmonella* spp, including *S*. *enteritidis* and *S. typhi* (typhoid fever), and *Shigella* spp, the agents of shigellosis (bacterial dysentery), *Campylobacter* spp (especially *C*. *jejuni*) and a variety of enteropathogenic (EPEC) *Escherichia coli* strains, and enterotoxigenic *Escherichia coli* (ETEC) strains. Diarrhea can also be caused by a variety of bacterial pathogens such as *Staphylococcus aureus, Clostridium perfringens, Clostridium difficile* or *Klebsiella* (WHO. Diarrhoeal Diseases. 2009). Diarrhea, in particular diarrhea caused by pathogenic bacteria, also is a common health problem among travellers who visit developing areas. Enterotoxigenic ETEC strains are the main agents of travellers' diarrhea.

Infection with several pathogens, such as Rotavirus, *Clostridium difficile, Salmonella, Campylobacter,* and *Shigella,* may lead to severe disease. Treatment may require extensive examination to determine the specific causative pathogen. The wide variety of bacterial and viral infections that may cause diarrhea complicates diagnosis, in particular in developing countries where there is little or no access to modern laboratory facilities. Another problem is the lack of diagnostic tools to differentiate infectious from non-infectious diarrhea. Acute diarrhea is currently diagnosed by bacteriological culture. However, such bacteriological cultures are expensive, and time-consuming and often don't result in identification of the causative agent, which results in delaying of treatment.

Probiotics, and in particular lactic acid bacteria, have been described as useful in the treatment of diarrhea of a variety of origins. However, probiotic studies on infectious, immune and inflammatory outcomes in humans are inconsistent, appear to be highly probiotic strain-dependent, and mainly focused on rotavirus diarrhea in children. For example, US 6,399,055 discloses the use of milk fermented with *L. casei* DN 114-001 in reducing the number, severity or duration of rotavirus-associated episodes of diarrhea in rats.

WO 00/54788 relates to compositions for regenerating intestinal microbiota in humans or animals, for instance in diarrhea. The composition comprises lactic acid bacteria, for instance *L. casei,* a calcium salt and an aluminium salt. In particular, use of such composition for regenerating intestinal flora following antibiotics induced diarrhea is disclosed. In WO 98/55131 use of *L. casei rhamnosus* LB21 for prevention and treatment of gastrointestinal disorders, among which diarrhea, is described. It is demonstrated that antibiotic-associated diarrhea, and growth of pathogenic bacteria is counteracted. LB21 is for instance added to a soured or fermented milk product. WO 2007/140621 relates to compositions comprising a mixture of probiotic bacteria including *Lactobacillus, Bifidobacterium, Propionibacterium* and *Streptococcus* useful in the prevention and treatment of gastrointestinal disorders. In WO 2006/088923 compositions comprising a probiotic and β-glucan are disclosed. The composition in particular contains the bacterium L. rhamnosus stain GG (LGG). The compositions may be milk products and are useful for treating gut disorders such as inflammatory bowel disease, stomach ulcers and diarrhea.

It is an object of the present invention to provide means and methods for the treatment of diarrhea which can be employed irrespective of the cause of the diarrhea. Said treatment preferably reduces the duration of an episode of diarrhea in an individual. The invention further contemplates providing a nutritional composition, in particular an infant's or children's drink, which can be used in the treatment of diarrhea.

The present inventors have surprisingly found that the combination of the probiotic strain *L. casei* CRL 431 and relatively high amount of calcium has a beneficial effect on the duration of an episode of both rotavirus-associated diarrhea and rotavirus-negative diarrhea. As demonstrated in the examples, in a randomised trail in Indonesian children, the mean duration of an episode of acute diarrhea was reduced by approximately half in children that received viable *L. casei* CRL 431 and calcium. As is demonstrated in table 4, the mean duration of a rotavirus-negative diarrheal episode was reduced by 49% in children which received a daily dose of 5x10⁸ CFU *L. casei* CRL 431 and 440 mg calcium as compared to children who received low calcium, i.e. a daily dose of about 50 mg, and no *L. casei* CRL 431. The mean duration of a rotavirus-associated diarrheal episode in children receiving *L. casei* and calcium was reduced by even 57%.

Accordingly, the invention provides a method for reducing the duration of an episode of diarrhea in an individual comprising administering to said individual viable *Lactobacillus casei* CRL 431 in an amount of at least 1x10⁶ CFU per 24 hours and calcium in an amount of at least 300 mg per 24 hours. Such method is herein also referred to as a method according to the invention.

In another aspect, the invention provides a nutritional composition comprising viable *Lactobacillus casei* CRL 431 in an amount of at least 1x10⁵ CFU per ml and calcium in an amount of at least 0.5 mg per ml. Such composition is herein also referred to as a nutritional composition according to the invention. Preferably said nutritional composition is a liquid composition.

Also provided is a combination of viable *Lactobacillus casei* CRL 431 and calcium for use in a method for reducing the duration of an episode of acute diarrhea in an individual, whereby said method comprises administering said *Lactobacillus casei* CRL 431 in an amount of at least 10⁶ CFU per 24 hours and said calcium in an amount of at least 300 mg per 24 hours. Further provided is a use of a combination of viable *Lactobacillus casei* CRL 431 and calcium for the preparation of a composition for reducing the duration of an episode of acute diarrhea in an individual, wherein said reducing comprises administering said *Lactobacillus casei* CRL 431 in an amount of at least 10⁶ CFU per 24 hours and said calcium in an amount of at least 300 mg per 24 hours.

Children of low socioeconomic status, for instance children in developing countries, are at a nutritional disadvantage, because they often do not consume enough food to meet the nutritional requirements for children of their age. In particular, children usually do not have enough calcium in their diets. Therefore, in developing countries where calcium intakes are characteristically low and the population has limited access to dairy products, dietary calcium deficiency is prevalent. Calcium is essential for strong bones and teeth. Calcium deficiency can lead to disorders like osteoporosis. In addition, in animal models dietary calcium improves intestinal resistance to infectious diarrhea due to *Salmonella enteritidis* and enterotoxigenic *E. coli* (ETEC) and to ETEC in a human proof-of-principle infection study in adults. However, the effect of dietary calcium on diarrhea in children is unknown.

Without wishing to be bound to any theory, it is believed that the reduction of the duration of a diarrheal episode is the result of the synergistic combined effect of the probiotic strain *L. casei* CRL431 and calcium because *L*. *casei* CRL431 and calcium are thought to exert their therapeutic effect via different mechanisms. These mechanisms may lead to synergistic effects. While calcium may adjust the environment within the gut towards a less favourite environment for pathogens, the *L casei* CRL431 may directly affect the growth of pathogens by competition for substrates and adhesion sites on the gut wall. In addition, *L. casei* CRL431 may indirectly affect growth of pathogens via stimulation of the gut's immune system and thus stimulation of host defence against pathogens.

Reduction of the duration of diarrhea contributes to health of an individual, in particular of children. Diarrhea has several detrimental effects on the human body, such as dehydration and electrolyte imbalances due to loss of fluids. Reducing the duration of diarrhea limits the magnitude of these effects, and may thus reduce diarrhea-associated morbidity and mortality. The consequences of diarrhea can be severe, in particular in children with low nutritional food intake and high infection pressure, such as in developing countries, and thus with impaired health status. Dehydration and electrolyte imbalances more readily lead to serious health problems in children with low nutritional status. Any reduction in the duration of diarrhea in such children, i.e. each additional diarrhea-free day, is important as it may help to prevent further deterioration of the child's delicate nutritional status and well-being.

The fact that compositions and methods according to the invention allow for decreasing the duration of both rotavirus-associated diarrhea and rotavirus-negative diarrhea has several advantages. Because a diarrheal episode is reduced irrespective of the cause of the diarrhea, it is not necessary to examine the cause of diarrhea first. Treatment can therefore be initiated as early as the appearance of the first symptoms of diarrhea. Diagnosis of a bacterial or viral infection that causes diarrhea, and of possible non-infectious causes is time-consuming. Diagnosis generally involves bacteriological culturing of stool samples which may take at least one or two days. Treatment in accordance with the invention can be initiated without delay, i.e. without waiting for determination of the cause of diarrhea. This results in a shorter duration of a diarrheal episode as compared to a situation wherein first the cause of diarrhea need to be examined before treatment of the diarrhea is started. Alternatively, in situations wherein the cause of the diarrhea is not, or not immediately, examined but treatment with antibiotics is started promptly upon the onset of the diarrhea, treatment in accordance with the invention obviates the need for antibiotics. In a preferred embodiment, a method according to the invention is thus initiated as soon as the first signs of diarrhea are apparent. Non-limiting examples of such signs are start of watery stools, abdominal pain and/or cramps and fever.

It is, however, also possible to initiate consumption of viable *L. casei* CRL 431 and calcium in the daily amounts indicated herein without signs of diarrhea being present. Consumption of *L. casei* CRL 431 and calcium in accordance with the invention is for instance sustained for a prolonged period of time, such as a month, two months, six months, a year, or even several years. As described herein below, in one aspect a nutritional composition according to the invention relates to a liquid food or dietary supplement for infants, which is particularly suitable for consumption during a prolonged period of time in which the child is exposed to many environmental pathogens. If *L. casei* CRL 431 and calcium are ingested for a prolonged period of time, the occurrence of diarrhea may even be prevented in addition to reducing the duration of a diarrheal episode.

In developing countries access to laboratory facilities where bacteriological cultures can be performed may be limited which further delays diagnosis, or even results in diagnosis being impossible to carry out. Even in developed countries, where laboratory facilities are not a limiting factor in analysis, examination of stool samples does not result in the identification of the causative agent of diarrhea in a substantial percentage of cases. Also bacteriological cultures are expensive. A method or nutritional composition according to the invention, which are useful in treating diarrhea caused by a variety of factors, obviates, or at least diminishes the need to diagnose the cause of diarrhea. This is in particular the case if the diarrhea is acute diarrhea. In a preferred embodiment, the invention therefore provides a method for reducing the duration of an episode of acute diarrhea. A nutritional composition according to the invention is also particularly suitable for reducing the duration of an episode of acute diarrhea.

Another advantage of a method and a nutritional composition according to the invention is that the same compounds, i.e. viable *L. casei* CRL 431 combined with calcium in the daily amounts indicated herein, can be used to reduce diarrhea irrespective whether it is a result of rotavirus infection or not. A method or composition according to the invention can be used for a wide variety of types of diarrhea. It is therefore not necessary to have different therapeutical or nutritional compositions available for each type of diarrhea. This is also in particular an important advantage in developing countries.

As used herein "diarrhea" refers to loose or liquid stools occurring at least three times per 24 hours. "Acute diarrhea" is defined as a suddenly occurring diarrhea with a maximum duration of 14 days. Determination of stool frequency is for instance as follows: stools are only counted when there is at least one hour between subsequent defecation moments.

An "episode of diarrhea" as used herein is defined as a period during which an individual experiences diarrhea as defined above, which period is considered to have ended on the last day of diarrhea if said day is followed by at least 2 diarrhea-free days.

"Rotavirus-associated diarrhea" relates to diarrhea which is caused by rotavirus infection and which is characterized by the presence of rotavirus in a diarrhea stool sample. Presence of rotavirus in stool is for instance detected as described in Iturriza-Gomara *et al.* (2009). As used herein "rotavirus-negative diarrhea" refers to diarrhea which is not associated with the presence of rotavirus because rotavirus can not be detected in a diarrhea stool sample.

"Bacteria-associated diarrhea" refers to diarrhea caused by infection with a pathogenic bacterium, such as, but not limited to, enterotoxigenic *E*. *coli* (ETEC), *Clostridium,* such as *C*. *difficile, Salmonella,* such as *S*. *enteriditis, S. typhi* and *S. paratyphi, Campylobacter,* such as *C. jejuni,* and *Shigella.*

As used herein, "treatment" refers to administration of calcium and L. casei CRL 431 according to the invention to an individual with the purpose to cure, alleviate, ameliorate, prevent, or reduce symptoms of diarrhea. Preferably, treatment of diarrhea reduces the duration of an episode of diarrhea in an individual.

"*L. casei* CRL 431" is the *L. paracasei* strain ssp. *paracasei* deposited under ATCC number 55544 and is available from manufacturer Chr. Hansen (Denmark). *L. casei* CRL 431 has been described as suitable for fermentation of milk. US 6,033,691 relates to a process for fermenting milk with a combination *of L. casei* deposited under ATCC number 55544 and *L*. *acidophilus* deposited under ATCC number 55543 with the purpose to increase the storage life of fermented milk. In addition, health benefits *of L. casei* CRL 431 in combination with other probiotics have been described. WO 2008/056983 discloses food products suitable for infants and children who are hypersensitive to components of ruminant milk. The food compositions comprise *L. casei* deposited under ATCC number 55544 and *B. lactis* deposited under ATCC number 27536.

The term "viable *L. casei* CRL 431 " as used herein refers to a live population *of L. casei* CRL 431 that is capable of replicating.

The term "CFU" is known to a skilled person and refers to colony forming units. It is a measure of the amount of viable bacteria. It is the minimum number of separable cells which is able to produce a visible colony of cells derived from the single progenitor cell.

The term "individual" as used herein is defined as a human or an animal, preferably a mammal. In a preferred embodiment of the invention an individual is a human. "Human" refers to infants, toddlers, children, teenagers, adults and elderly people, both men and women. In a preferred embodiment, "individual" or "human" refers to an infant in the age of 0-10 years, preferably to an infant in the age of 0-6 years.

Ingestion of between 10⁶ and 10¹⁰ CFU (colony forming units) per 24 hours *of L. casei* CRL 431 and between 330 and 800 mg of calcium was typically found to be sufficient to achieve the desired effect of reducing the duration of an episode of diarrhea. Thus, a method according to the invention preferably comprises administration of at least 10⁶ CFU *of L. casei* CRL 431 and at least 300 mg calcium per 24 hours. Preferably, said dose *of L. casei* CRL 431 is at least 5x10⁶ CFU, more preferably at least 10⁷ CFU, more preferably at least 5x10⁷, more preferably at least 10⁸ CFU per 24 hours.

The amount of calcium administered is preferably at least 330 mg per 24 hours, more preferably at least 350 mg, more preferably at least 375 mg, more preferably at least 400 mg, more preferably at least 425 mg per 24 hours, based on the weight of Ca²⁺ when present in a nutritional composition. Typical examples of calcium sources are calcium-caseinate and calcium phosphate. The indicated amount of calcium is then based on the weight of the free Ca²⁺ after dissolving of these calcium compounds in a suitable liquid, such as water or a milk product. In an exemplary embodiment of the invention, about 5x10⁸ CFU *of L. casei* CRL 431 and about 440 mg of calcium are administered to an individual per 24 hours.

The amount *of L. casei* CRL 431 in a nutritional composition of the invention may vary, and depends amongst others on the volume of the composition that is consumed daily. Typically, a daily intake of a liquid nutritional composition according to the invention is between 100 and 700 ml.

As detailed herein above, a preferred dose of *L. casei* CRL 431 is between 10⁶, preferably 10⁷, and 10¹⁰ CFU per 24 hours. A nutritional composition according to the invention may therefore comprise a range as wide as about 10³ CFU *of L. casei* CRL 431 per ml (for a consumption of about 700 ml and a daily dose of 10⁶ CFU *of L. casei* CRL 431) to 10⁸ CFU *of L. casei* CRL 431 per ml (for a consumption of about 100 ml and a daily dose of 10¹⁰ CFU of *L. casei* CRL 431). Said nutritional composition may further comprise a range as wide as about 0.4 mg of calcium per ml (e.g. for a consumption of about 700 ml and a daily dose of 300 mg of calcium) to 7 mg of calcium per ml (e.g. for a consumption of about 100 ml and a daily dose of 700 mg of calcium). Preferably, however, a nutritional composition according to the invention comprises between 10⁵ and 10⁷ CFU per ml of viable *L. casei* CRL 431 and between 0.6 and 4 mg/ml of calcium.

The source of calcium present in a nutritional composition can be provided in any form. It is possible to use soluble salts of calcium such as calcium chloride, calcium monophosphate, calcium chloride, calcium citrate, calcium ascorbate, calcium acetate, calcium gluconate, dicalcium phosphate, calcium tartarate. Other possible sources include, but are not limited to, calcium lactate and calcium caseinate, or insoluble calcium salts, such as calcium triphosphate, calcium sulphate and calcium carbonate. Provided is thus a nutritional composition according to the invention wherein calcium is present in the form of soluble salts of calcium and/or in the form of insoluble salts of calcium, and/or in the form of calcium lactate and/or calcium caseinate. Preferred calcium sources are calcium lactate, calcium caseinate, monocalcium phosphate, dicalcium phosphate and/or calcium triphosphate.

The daily amount *of L. casei* CRL 431 (at least 10⁶, preferably at least 1x10⁷, CFU) and calcium (at least 300 mg, preferably at least 330 mg) may be consumed over several portions throughout the day. In one embodiment, a dose of 2.5x10⁸ CFU *of L. casei* CRL 431 and 220 mg of calcium is consumed twice daily to reach a total dose of 5x10⁸ CFU *of L. casei* CRL 431 and 440 mg calcium per 24 hours. In another embodiment, five times a dose of 2x10⁷ CFU *of L. casei* CRL 431 and 80 mg of calcium is consumed to reach a total dose of 10⁸ CFU *of L. casei* CRL 431 and 400 mg calcium per 24 hours. Alternatively, the daily amount of *L. casei* CRL 431 and calcium is consumed in a single dose. As described above, typically a daily intake of a liquid nutritional composition according to the invention is between 100 and 700 ml, such as 200, 250, 300, 330, 360, 400, 450 or 500 ml.

In an exemplary embodiment, the daily dose of at least 10⁶ CFU of viable *L. casei* CRL 431 and at least 300 mg calcium is consumed in two doses of a liquid composition of about 180 ml per 24 hours, whereby said liquid composition either comprises viable *L. casei* CRL 431 and calcium, or whereby said liquid composition comprises said calcium and is consumed via a straw coated with viable *L. casei* CRL 431. Typically, if a liquid nutritional composition, such as non-fermented milk, is consumed via a straw coated with viable *L. casei* CRL 431, preferably in vegetable oil, approximately all of the *L*. *casei* present inside the straw will be ingested. Thus, for a daily dose of at least 10⁶ CFU of viable *L. casei* CRL 431 a coated straw comprises at least 10⁶ CFU of viable *L. casei* CRL 431, if said daily dose *of L. casei* CRL 431 is consumed with a single intake.

*L. casei* CRL 431 is a probiotic bacterium. The term "probiotic" refers to a microorganism which confers a health benefit to an individual after administration. In a method according to the invention probiotic bacteria other than *L. casei* CRL 431 may be used. In addition, a nutritional composition according to the invention may comprise probiotic bacteria other than *L. casei* CRL 431. Probiotic bacteria are known to the skilled person. Non-limiting examples of such probiotic bacteria include lactobacilli, e.g. *Lactobacillus paracasei, Lactobacillus casei, Lactobacillus reuteri, Lactobacillus helveticus, Lactobacillus rhamnosus, Lactobacillus johnsonii, lactobacillus acidophilus, Lactobacillus salivarius,* and/or Bifidobacteria, e.g. *Bifidobacterium animalis, Bifidobacterium lactis, Bifidobacterium bifidum, Bifidobacterium longum, Bifidobacterium breve, Bifidobacterium infantis.* In a preferred embodiment, a liquid composition according to the invention further comprises *Bifidobacterium animalis* subsp. *lactis.*

The present inventors for the first time identified that the combination of *L. casei* CRL 431 and high calcium is effective in reducing the duration of a diarrhea episode, irrespective of the cause of diarrhea. Therefore, in a preferred embodiment, *L. casei* CRL 431 is the main bacterial agent used in a method according to the invention or present in a nutritional composition according to the invention. Thus, *L. casei* CRL 431 and calcium are the essential components administered to an individual in a method according to the invention. With "main bacterial agent" it is meant that *L. casei* CRL 431 constitutes at least 50%, in terms of CFU, of the total amount of bacteria administered to an individual or present in a nutritional composition according to the invention. Preferably, *L. casei* CRL 431 constitutes at least 60% of the total amount of bacteria, more preferably at least 70%, more preferably at least 75%, more preferably at least 80%, more preferably at least 85%, more preferably at least 90%, most preferably at least 95%. In another preferred embodiment, *L. casei* CRL 431 is the only bacterial agent used in a method according to the invention or present in a nutritional composition according to the invention.

In a preferred embodiment, viable *L. casei* CRL 431 and calcium are administered simultaneously to an individual. With "simultaneously" it is meant that said *L. casei* CRL 431 and said calcium are administered at the same time or within a limited period of time from each other, for instance within 1, 2, 3, 4, 5, 6, 7, 8, 9 or 10 minutes. Simultaneous administration is for instance achieved by administering a single nutritional composition according to the invention comprising both the high calcium and viable *L. casei* CRL 431 to an individual. Alternatively, a first composition comprising viable *L. casei* CRL 431 and a second composition comprising calcium are administered simultaneously. In yet another embodiment, simultaneous administration is achieved by drinking a liquid nutritional composition comprising calcium through a straw coated with viable *L. casei* CRL 431.

In one aspect of the invention, the viable *L. casei* CRL 431 and calcium are present in separate containers. For instance, calcium is present in one container, either as particulate calcium, such as in the form of a calcium salt, calcium caseinate or calcium lactate, or in a liquid composition, such as water or non-fermented milk, and the *L. casei* CRL 431 is present in a second container, for instance in the form of a dry powder. The viable *L. casei* CRL 431 and calcium are preferably combined prior to ingestion, for instance by mixing the content of the two separate containers, or during ingestion, for instance by consumption of a drink comprising calcium through a straw comprising viable *L. casei* CRL 431. The invention thus provides a kit of parts comprising a first container comprising a liquid nutritional composition comprising calcium in an amount of at least 0.6 mg per ml, preferably between 0.6 and 4 mg/ml of calcium, and a second container comprising viable *L. casei* CRL 431 in an amount of at least 10⁶, preferably at least 10⁷ CFU, more preferably at least 10⁸ CFU.

In one embodiment, viable *L. casei* CRL 431 and calcium are administered to an individual via a liquid nutritional composition, e.g. non-fermented milk, which comprises calcium but not *L. casei* CRL 431, whereby the composition is ingested via straws internally coated with viable *L. casei* CRL 431. Provided is therefore a kit of parts according to the invention, wherein said second container comprises a straw coated internally with viable *Lactobacillus casei* CRL 431. If a liquid composition is ingested via such a straw internally coated with viable *L. casei* CRL 431, the bacteria are released from the straw and are carried along and ingested with the liquid. For instance straws are coated on the inside with viable *L. casei* CRL 431 in vegetable oil. An example of a suitable vegetable oil is rapeseed oil.

In another embodiment, the invention provides a kit of parts according to the invention, wherein said second container is a closed container, such as a sachet or capsule, comprising the viable *L. casei* CRL 431, for instance in the form of a dry powder. The composition comprising calcium and the viable *L. casei* CRL 431 are for instance combined before administration to an individual by mixing the contents of the first and the second container.

Preferred nutritional compositions according to the invention are liquid nutritional compositions. These are preferably selected from the group consisting of dairy drinks, e.g. milk, skim milk, reduced-fat milk and buttermilk, products that are derived from dairy like yoghurt and yoghurt drinks, and calcium-fortified fruit juices. Yoghurt and buttermilk are dairy products that have been fermented with lactic acid bacteria such as *Lactobacillus, Lactococcus,* and *Leuconostoc.* The general pathway for fermentation of milk involves the production of lactic acid from the sugar lactose in the milk. In a preferred embodiment, a nutritional composition according to the invention is a non-fermented milk product, such as pasteurized milk or microfiltered milk.

Pasteurization refers to various forms of elevated temperature treatment, with the temperature and time of the treatment determining the result. Thus, regular pasteurized "fresh" milk that can be stored for a few weeks, if refrigerated, is treated by a so-called HTST process ("high temperature/short time). This regular pasteurization generally refers to heating at about 72°C for 15-20 seconds. Alternatively, milk products are so pasteurized to be suited for non-refrigerated storage (referred to, in the context of the invention, as 'long-life pasteurization'). Pasteurization is generally done in a plurality of subsequent heat treatments or in a single heat treatment, normally at a temperature in the range of from 60°C to 110°C. The heat treatment process of pasteurization is not only dependent on the treated material but also on other conservation parameters like pH, storage temperature in stock, and water activity.

A preferred example of heat treatment is the UHT treatment (Ultra High Temperature). This is also referred to as ultra-pasteurization. Preferred treatments are those referred to in Tetra Pak Diary Processing Handbook (for dairy products) and Steven Nagy, Fruit Juice Processing Technology. Particularly suitable UHT treatments are heating at 135-150°C for 15-4 seconds (diary) and in the case of fruit juice for 15-60 seconds at 95-90°C. A further preferred treatment, notably for dairy products, is at a temperature of 138°C or 143°C for a fraction of a second. Another 'long-life pasteurization' treatment, i.e. a pasteurization treatment resulting in long-life products, is HHST (higher heat/shorter term) which lies in between HTST and UHT in terms of time and temperature. Irrespective of the exact treatment chosen for providing a long-life, the invention preferably pertains to compositions that can be stored outside of a refrigerator, and particularly products that have undergone a temperature treatment to achieve this status.

Microfiltration is a process whereby milk is forced through a microfilter, using membranes with a pore size of typically 0.1 to 0.2 µm, that removes most of the bacteria. Using microfiltration a higher number of microorganism may be removed as compared to pasteurization and a longer shelf life may be obtained. Microfiltration can therefore also be performed prior to pasteurization, allowing for an extended shelf-life of milk products prepared with standard pasteurization techniques.

Milk used for nutritional compositions provided by the invention can, in principle, be from any ruminant animal. This is preferably milk from cattle, and particularly cow, but in addition to cattle, the following animals provide milk that can be used for nutritional composition according to the invention: camels, goats, deer, sheep, water buffalo, yaks, and moose. Preferably, the milk used in the invention is cow's milk, most preferably the low-fat fraction of the raw milk, called skim milk. The nutritional product can also be on the basis of dairy alternatives, such as soy. The calcium content of cow's milk is approximately 1.2 mg/ml and that of yoghurt prepared from cow's milk 1.0 mg/ml. Thus, for example, if the nutritional composition used in accordance with the invention is a cow's milk product, approximately 250 ml per 24 hours of such milk product is sufficient to provide the amount of at least 330 mg per 24 hours of calcium. Said milk product is for instance supplemented with viable *L. casei* CRL 431 in an amount of at least 10⁷ CFU. Alternatively, said milk product is consumed via a straw coated with at least 10⁷ CFU of viable *L. casei* CRL 431. Milk from animals other than cow may contain less calcium, which, as opposed to cow's milk, is not sufficient to provide the minimal amount of calcium. Milk products derived from milk of such animals are therefore preferably supplemented with additional calcium until the desired amount of calcium is present in said milk product. Also, soy milk contains less calcium as compared to cow's milk, approximately 0.2 mg/ml. Therefore, if a nutritional composition according to the invention comprises a soy milk product, the composition is preferably supplemented either with both calcium and viable *L. casei* CRL 431, or with calcium and is consumed via a straw coated with at least 10⁶ CFU, preferably at least 10⁷ CFU, of viable *L. casei* CRL 431, to achieve the daily amounts required according to the invention.

Nutritional compositions according to the invention are suitable for use for infants, toddlers, children, teenagers, adults and elderly people, both men and women In a particular aspect, the nutritional composition relates to a liquid food or dietary supplement for infants, i.e. an infant or toddler formula. In one embodiment, therefore an infant formula is provided which comprises, in addition to the viable *L. casei* CRL 431 and calcium, a carbohydrate source, a lipid source and a protein source. As used herein, the term "infant formula" of "toddler formula" refers to a composition that satisfies total nutrient requirements for an infant or toddler, respectively, thus being a substitute for human breast milk. Infant (baby) formula is generally for use, in addition to or instead of human breast milk, with infants up to 18 months old. Toddler formula generally refers to follow-on formula for children of 18-48 months. In a specific embodiment, it relates to a complete infant food up to the age of 6 months. Another specific form relates to an infant food for infants of an age from 6 months to 3 years. In another aspect, the invention relates to a milk product for children in the age group of 3 to 6 years or in the age of 3 to 10 years. Preferably, in a method of the invention viable *L. casei* CRL 431 and calcium is administered to an infant in the age group of 0-6 years.

In a specific embodiment, the invention provides a liquid nutritional composition or dry powder according to the invention suitable for use as a dietary supplement for children who generally have a low daily intake of calcium, i.e. less than 75% of the RDA (recommended dietary allowance, the average daily level of intake sufficient to meet the nutrient requirements of healthy individuals) of 500 mg/day for infants under the age of 6 months, 600 mg/day for infants from 6-12 months, 400 mg/day for children aged 1-3 years, and 450 mg/day for children aged 4-6 years (WHO, Feeding and nutrition of infants and young children, WHO Regional Publications, European Series, No. 87, updated 2003).

A nutritional composition, such as infant or toddler formula, or a liquid milk product, provided in accordance with the invention, may comprise any further conventional ingredient. For instance one or more of the following compounds may be added: carbohydrates, such as oligosaccharides, lipids and ingredients such as vitamins, amino acids, minerals, taurine, carnitine, nucleotides and polyamines, a protein source, such as whey proteins, α-lactalbumin, lactoferrin and vegetable proteins, as from soybean or wheat, flavouring agents, colouring agents and antioxidants such as BHT, ascorbyl palmitate, vitamin E, α- and β-carotene, lutein, zeaxanthin, lycopene and lecithin. The lipids are preferably of vegetable origin. In addition, the nutritional composition may be enriched with polyunsaturated fatty acids, such as gamma-linolenic acid, dihomo-gamma-linolenic acid, arachidonic acid, stearidonic acid, eicosapentaenoic acid, docosahexaenoic acid and docosapentaenoic acid. Flavours include, but not limited to, chocolate, vanilla, strawberry, raspberry, peach, blueberry, cherry and lemon. In an exemplary embodiment, the nutritional composition is a non-fermented chocolate flavoured liquid milk product.

As herein described before, a nutritional composition according to the invention is particularly suitable for children in developing countries because in particular in these children diarrhea is the main cause of morbidity and mortality. In addition, children in developing countries often do not have sufficient amounts of calcium in their diet. In developing countries, in particular in Asian, African and Native American populations, the incidence of lactose intolerance is high. Lactose intolerance is the human body's inability to digest and absorb the milk sugar lactose. It is caused by a lack of lactase, the enzyme required to break down lactose in the digestive system, and results in symptoms including abdominal pain, diarrhea, nausea and acid reflux. The frequency of decreased lactase activity ranges from 5% in northern Europe to more than 90% in some African and Asian countries.

In one embodiment, therefore, a nutritional composition according to the invention has a low lactose content. Preferably, the daily lactose intake of a lactose intolerant individual is not more than 12 grams. Therefore, in one embodiment a nutritional composition according to the invention is formulated to meet the maximum lactose intake of a lactose-intolerant subject. For example, it comprises at most 4 grams of lactose per 100 ml, preferably at most 3 grams of lactose per 100 ml, more preferably at most 2 grams of lactose per 100 ml. Lactose contents of up to 1 gram of lactose per 100 ml, preferably at most 0.5 grams of lactose per 100 ml are also encompassed. In one embodiment, a nutritional composition according to the invention comprises about 0.1 gram of lactose per 100 ml.

A liquid nutritional composition according to the invention, for instance a non-fermented milk product, may be concentrated to a dry product. Such dry product can be reconstituted, for instance with water or another suitable liquid, to a liquid product ready for consumption. The invention therefore also provides a dry powder comprising calcium and viable *L. casei* CRL 431, which upon reconstitution with water, provides a nutritional composition according to the invention. Such dry powder is herein also referred to as a dry powder according to the invention. The liquid used for reconstitution of a dry product preferably has a temperature at which at least part of the *L. casei* CRL 431 remains viable. Typically said temperature is not more than 100°C, preferably not more than 90°C. Infant formula for infants less than 1 year of age are typically heated to at least 75°C. In a particular embodiment, the temperature of the liquid is therefore not more than 80°C, such as not more than 75°C. Nutritional compositions for children more than 1 year of age do not need to be heated. In another aspect, the temperature of a liquid used for reconstitution of a dry product according to the invention, preferably water, is therefore not more than 60°C, preferably not more than 50°C. In a particular aspect, said temperature is not more than approximately 37°C.

Dry products have the advantage that they have a longer shelf life due to the reduced level or even lack of water. In addition, dry products are less heavy, and have a smaller volume so that transportation is easier. Conventional drying techniques may denature a considerable amount of the proteins present in milk products. In addition, it is essential that the *L. casei* CRL 431 present in the liquid product remains viable throughout the concentration process, as the bacteria will only exert their advantageous function in diarrhea if it is consumed in viable form. Therefore, if a concentration method is employed it is desirable to use a mild concentration method, such that less than 25wt% of the protein in the composition is denatured in the dried product, and the *L. casei* CRL 431 remains viable. Suitable drying steps are spray drying, drying in the presence of surface active components, gas injection, drying with super critical CO₂, freeze drying, forward osmosis, reverse osmosis, membrane distillation, thin-film spinning cone evaporator, and scraped film evaporation. Whether or not the *L. casei* CRL 431 remains viable during drying depends on several factors, such as the presence of other components in the liquid product and the temperature and duration of heating. For instance, heating above 50°C may result in a decrease of the amount of viable organisms in the dried product as compared to the amount of viable organisms before drying. Preferred methods are spray drying and freeze drying because the temperature of the product remains relatively low during these processes. The moisture content of a dry powder according to the invention is preferably less than 5%. Said dry powder is preferably soluble in water.

A nutritional composition or dry powder according to the invention can be advantageously used to reduce the duration of an episode of diarrhea in an individual. The invention thus provides a nutritional composition or dry powder according to the invention for use as a medicament. Further provided is a nutritional composition or dry powder according to the invention for use in a method for reducing the duration of an episode of diarrhea in an individual, whereby said method preferably comprises administering said *L. casei* CRL 431 in an amount of at least 10⁶ CFU, preferably at least 10⁷ CFU, per 24 hours and said calcium in an amount of at least 300 mg, preferably at least 350 mg, per 24 hours. Also provided is a method according to the invention comprising administering to an individual in need thereof a nutritional composition according to the invention.

A nutritional composition according to the invention may further contains at least one prebiotic substance. Prebiotic substances are indigestible food ingredients that beneficially affect the host by selectively stimulating the growth and/or activity of one or more bacteria in the intestines, either beneficial gut bacteria or ingested probiotic microorganisms, and thus exerts a health benefit in the host. The best-known prebiotic substances are indigestible oligosaccharides. Examples of suitable prebiotic substances are fructo- and/or galacto-oligosaccharides, with short or long chains, polysaccharides, and other prebiotic substances that contain fructose, xylose, soy-oligosaccharides, galactose, glucose and/or mannose, inulin, fucose-containing oligosaccharides, branched (oligo)saccharides, beta-glycans, carob bean flour, gums, which may or may not be hydrolyzed, pectins, (fuco)sialyloligosaccharides, sialyllactose, galactans with short or long chains, fibers, protein hydrolysates, and nucleotides. Regular amounts of these substances are known to the skilled person and will preferably be about up to 0.1-10 grams, preferably 1-5 grams, or 2-3 grams a day dependent on the prebiotic substances.

The invention is further explained in the following examples. These examples do not limit the scope of the invention, but merely serve to clarify the invention.

### References

Bovee-Oudenhoven IM, Lettink-Wissink ML, Van Doesburg W, Witteman BJ, Van Der Meer R. Diarrhea caused by enterotoxigenic Escherichia coli infection of humans is inhibited by dietary calcium. Gastroenterology 2003;125:469-76.
Gibson RS. Principles of Nutritional Assessment, Second Edition, Oxford University Press, Oxford, 2005.
Iturriza-Gomara M, Elliot AJ, Dockery C, Fleming DM, Gray JJ. Structured surveillance of infectious intestinal disease in pre-school children in the community: 'The Nappy Study'. Epidemiol Infect 2009;137:922-31.
Lewis SJ, Heaton KW. Stool form scale as a useful guide to intestinal transit time. Scand J Gastroenterol 1997;32:920-4.
Shastri YM, Bergis D, Povse N, Schäfer V, Shastri S, Weindel M, Ackermann H, Stein J. Prospective multicenter study evaluating fecal calprotectin in adult acute bacterial diarrhea. Am J Med. 2008; 121(12): 1099-106.
WHO. Initiative for Vaccine Research (IVR). Diarrhoeal Diseases (Updated February 2009).
WHO. The treatment of diarrhoea: a manual for physicians and other senior health workers, WHO/CDR/95 • 3. Geneva: World Health Organization, 4th rev, 2005.

### Experimental section

### Subjects and Methods

A randomized, double-blind, placebo-controlled trial was conducted in fifteen urban communities representing non-flooding and flooding areas of East Jakarta, Indonesia. These areas were selected on the basis of a number of criteria: high population density, low socioeconomic status, and high prevalence of diarrhea and underweight in under-five children, based on routine data from the primary health care system and local government. Children aged 1-6 years were selected from community registry for the first screening phase to assess eligibility based on the following inclusion criteria: apparently healthy, not being breastfed and regular consumption of milk but calcium intake <375 mg/day (<75% of the recommended daily allowance for this age). In the second phase, registered physicians interviewed mothers and examined the children to confirm their health status and to check the following exclusion criteria: symptoms of chronic/congenital diseases and disabilities, pulmonary tuberculosis, history of allergy, diarrhea on admission, antibiotics use within 2 weeks prior to study start, severe wasting (<-3 SD of weight-for-height z score), total calcium intake >375 mg/day according to a validated food-frequency questionnaire, not capable or willing to drink liquid milk with a straw in a 2-day acceptance test, and/or showing allergy or intolerance to the products, sibling of an already included child (except a twin sibling).

Children were randomly assigned to receive low lactose milk: low lactose milk (0.01 g/L) with a low calcium content (≈50 mg Ca/day, LC); the same milk with a regular calcium content (≈440 mg Ca/day, RC); or RC plus *Lactobacillus casei* 431 (5x10⁸ CFU/day, casei). Milk was sweetened, chocolate-flavored, ambient stable (UHT sterilized), and packed in tetra packs (Frisian Flag Indonesia, Jakarta). Milk was consumed with straws coated inside with probiotics in vegetable oil or just the oil drop as placebo (BioGaia AB, Sweden). *L. casei* 431 (Christian Hanssen, Denmark) was coated into straws. The different milk drinks and straws were indistinguishable for the investigators and participants. The composition of the milks and straws is described in Table 1.

**Table 1. Composition of Low Calcium (LC) and Regular Calcium (RC) Milk and Probiotic Straws**

| | **Composition** | **LC** | **RC** | **RC + Casei** |
|---|---|---|---|---|
| **UHT Milk (per 100 ml)** | | | | |
| | Energy,kcal | 93.8 | 98.0 | 98.0 |
| | Fat, g^{a} | 3.5 | 3.9 | 3.9 |
| | Protein, g^{a} | 3.9 | 3.8 | 3.8 |
| | Total carbohydrate, g^{a} | 11.7 | 12.0 | 12.0 |
| | Lactose, g | 0.07 | 0.09 | 0.09 |
| | Vitamin A, µg | 32 | 30 | 30 |
| | Calcium, mg^{a} | 15 | 129 | 129 |
| | Phosphor, mg | 32 | 77 | 77 |
| | Magnesium, mg | 6 | 6 | 6 |
| | Iron, mg | 0.30 | 0.30 | 0.30 |
| | Zinc, mg | 0.14 | 0.14 | 0.14 |

| **Straw probiotic (CFU/day)** | | | | |
|---|---|---|---|---|
| | *Lactobacillus casei* 431 | - | - | 5x10⁸ |

| | | | | |
|---|---|---|---|---|
| Note: CFU indicates colony-forming units. ^{a}Based on chemical analyses. | | | | |

Milk and straws were stored cooled (<10°C) at all times until delivery. Viability of the probiotics was checked each month by selective plating. Fieldworkers distributed milk and straws twice a week to the parents, who were instructed to store products refrigerated and prevent sun exposure. Parents who did not have a refrigerator, obtained the products from the fieldworkers' house on daily basis and/or children consumed the products directly at the fieldworkers' house.

Mothers were instructed to provide the children 180 ml of milk twice daily (not with a meal) using the straws provided. Mothers were requested to maintain the child's habitual diet, but to exclude probiotic, prebiotic or high calcium foods/drinks, other than the supplied ones. During diarrheal episodes, children continued or restarted drinking milk as soon as possible but after being rehydrated with Oral Rehydration Solution (ORS) according to WHO guidelines (WHO/CDR/95 • 3. Geneva: World Health Organization, 4th rev, 2005).

### Data Collection

Fieldworkers collected fecal samples before and at the end of intervention (last day of supplement intake), and during diarrheal episodes. Diarrheal samples were collected from onset of diarrhea until maximally 3 days later if diarrhea was still present. Stools contaminated with urine or fallen into the toilet or the child's underwear were discarded. Collected stools were directly stored and transported in cool boxes with ice packs to a special freezer (-20°C) at the fieldworkers' house (max. one week) until storage in a freezer at SEAMEO RECFON laboratory (-70°C).

Before and at the end of intervention, non-fasting venous blood was drawn in the morning by trained phlebotomists. At baseline, fieldworkers interviewed mother (or caregivers) on the family's socioeconomic and demographic characteristics, their environment (i.e sewage, domestic disposal), maternal hygiene (handwashing, food preparation hygiene), and caring (i.e responsible person taking care of child) and nutrition practices for the children and the child's general health. Prior to blood collection, a study physician examined health status of children and fieldworkers performed anthropometric measurements.

### Follow Up Observation for Diarrhea

Mothers recorded daily defecation pattern (time, frequency and visual appearance of the stools, Gibson RS. 2005). Feces was graded as 1 (normal), 2 (loose), 3 (semi liquid), and 4 (liquid) on a structured form during the trial as previously described (Lewis SJ and Heaton KW. 1997). Children with signs of dehydration, persistent diarrhea, severe vomiting or high fever were referred to the study physician and received medical care according to WHO guidelines (WHO/CDR/95 • 3. Geneva: World Health Organization, 4th rev. 2005). Records of fieldworker were randomly checked by the field supervisor. Final Vesikari scores in the trial database were entered by field supervisors and verified by the study physicians. In addition, the occurrences of all other adverse events were recorded by fieldworkers on a structured and pretested form. Final diarrhea and adverse events diagnoses and recording in the trial database were verified by the study physicians. All adverse events were recorded using ICD-10 codes.⁴⁰ Severity and likelihood of relation to the interventions were scored by the study physician and continuously monitored by the data-safety monitoring board (DSMB). All children were followed-up until the endline measurement. An independent expert monitored trial conduct and adherence to the protocol.

### Laboratory Measurements

Collected stools were freeze-dried and analyzed for calcium (base- and endline samples) and rotavirus (diarrheal samples) using previously described methods (Bovee-Oudenhoven IM, *et al*. 2003; and Iturriza-Gomara M, *et al.* 2009).

### Assessments of Efficacy and Safety

The outcomes presented in this paper were diarrhea severity as assessed by the Vesikari score and the above mentioned fecal biomarkers, the duration of diarrheal episodes and the presence of rotavirus in diarrheal samples.

Diarrhea was defined according to the strict WHO definition (≥3 loose/liquid stools in 24 h). For determination of stool frequency, stools were only counted when there was at least one hour between subsequent defecation moments. An episode was considered to have ended on the last day of diarrhea followed by 2 diarrhea-free days. The number of episodes is the sum of all diarrheal episodes that occurred during the intervention period. The duration of a diarrheal episode was defined as number of days from first until last excretion of the loose or liquid stool that is not followed by another abnormal stool in each of the episodes. The cumulative (total) duration of diarrhea was the sum of all diarrheal days per child per 6-month of intervention period.

The severity of diarrhea was assessed by the above-mentioned fecal biomarkers and by using the Vesikari scoring system. The latter was slightly adjusted to better fit with the community situation and study population. The Original 20-point numerical score includes the assessment of duration of diarrhea (3 points), maximal frequency of diarrhea in 24-h (3 points), duration of vomiting in days (3 points), maximal frequency of vomiting in 24 h (4 points), fever in degrees Celcius (3 points), percentage of dehydration (3 points) and rehydration given or hospitalization (2 points). We graded the severity by using a slightly modified 18-point Vesikari scale and asked a qualitative report of mothers for fever (yes or no) instead of rectal temperature. Also, the percent dehydration was replaced by estimated degree of dehydration since this variable is not easily assessed in a community setting. We defined mild to moderate disease as scores of <11 and severe disease as scores of ≥ 11.

### Statistical Methods and Data Analysis

Intention-to-treat (ITT) analysis was performed for all outcomes and for all eligible children who were randomly allocated to treatment and had consumed the intervention products at least once. Analyses were carried out according to a predefined data analysis protocol.

For all outcomes, double data entry was applied. Chi-square test was used for comparison of categorical variables between groups and Fisher exact test was used when one cell had an expected count <5. Student t-test was used to identify differences in quantitative normally distributed data between predefined groups (between LC and RC; RC and casei). Mann-Whitney U test was applied to identify differences in quantitative non-normally distributed data between predefined groups. We used PASW Statistic 17.0.3 for windows (SPSS, Chicago, IL 2009) for the analyses.

Disease incidence was the number of episodes divided by child-years of observation. For count outcomes (episodes, duration and vesikari score) we used the Negative binomial model in case of excess zeros and over-dispersion, to estimate the relative risk (RR) and 95% Confidence Intervals (CI) between groups.

### Results

At admission, all study groups were comparable regarding socio-demographic characteristics (including environment, and maternal hygiene and caring), health and hematology status, and habitual dietary intake (Table 2). Changes of habitual dietary intake between baseline and endline were not significantly different between groups. The compliance to study products use was high (94%) and similar among groups. Analysis of both probiotic strain viabilities indicated a decrease of less than 10% of CFU over the intervention period. The incidence and mean number of episodes according to the WHO definition of severe diarrhea were not significantly different among groups (Table 3). Analyses of the diarrheal children (n=100, together having 152 episodes) showed that the cumulative duration of diarrheal episodes tended to be shorter (36%) in the RC + casei group (95% CI 0.39-1.03; P=0.07). Calcium did not affect duration of diarrheal episodes. Six episodes of persistent diarrhea (duration of diarrhea>14 days) were recorded: 1 in RC, 3 in LC and 1 in the RC + casei group). It should be noted that, except for one episode, almost all diarrhea in our study was categorized as mild based on the symptom severity scores of the modified Vesikari scale. Vesikari scores were not different between the dietary treatment groups RC and LC (RR 1.02, 95% CI 0.79-1.32), RC + casei and RC (RR 1.01, 95% CI 0.78-1.31) (Table 3).

The prevalence of rotavirus in collected diarrheal samples was 28% in RC, 31% in LC, and 33% in RC + casei (Table 4). Diarrhea duration of rotavirus-positive episodes were significantly longer in LC group (LC *υs* RC RR 2.48 95% CI 1.26-4.90; P = .01). In rotavirus-negative episodes, diarrhea duration was shorter in the RC + casei group (RC + casei *υs* RC RR 0.52 95% CI 0.28-0.96; P = .04). Severity of diarrhea, as assessed by the Vesikari score, did not differ between rotavirus-negative and rotavirus-positive episodes (Table 4).

**Table 2. Baseline characteristics of the Indonesian children in three intervention groups**

| **Characteristic** | **LC (n=124)** | **RC (n=126)** | **RC + Casei (n=120)** |
|---|---|---|---|
| **Areas** | | | |
| Flooding | 81 (65%) | 82 (65%) | 78 (65%) |
| Non flooding | 43 (35%) | 44 (35%) | 42 (35%) |
| **Age** (months) | 59.3 (14.3) | 58.9 (14.2) | 60.3 (13.7) |
| **Sex** (male) | 67 (54%) | 68 (54%) | 66 (55%) |
| Household | | | |
| expenditure | 189 (97) | 194 (139) | 159 (69) |
| (US$ per months) ^{a} | | | |
| Mother's education <6 | 43 (36%) | 43 (35%) | 52 (42%) |
| years | | | |

| **Health status** | | | |
|---|---|---|---|
| Diarrhea 2 wk prior to | 20 (16%) | 13 (10%) | 24 (20%) |
| study | | | |
| ARI 2 wk prior to study^{b} | 48 (39%) | 51 (41%) | 52 (43%) |

| **Hematology profile** | | | |
|---|---|---|---|
| hs-CRP (mg/L) | 0.8 (0.2, 1.8) | 0.8 (0.3, 2.9) | 0.8 (0.3, 2.5) |
| AGP (g/L) | 0.8 (0.7, 0.9) | 0.8 (0.7, 1.0) | 0.8 (0.7, 1.0) |
| Anemia | 24 (19%) | 33 (26%) | 24 (20%) |

| **Nutritional status,** | | | |
|---|---|---|---|
| mean (SD) | | | |
| Weight-for-age | -1.26 (1.2) | -1.15 (1.1) | -1.27 (1.1) |
| Height-for-age | -1.47 (1.1) | -1.39 (1.0) | -1.53 (1.0) |
| Weight-for-height | -0.65 (0.9) | -0.58 (1.0) | -0.51 (1.0) |
| Fecal calcium (mg/g) | 7.6 (4.7, 11.1) | 7.5 (4.8, 10.4) | 6.6 (4.8, 9.3) |

| **Habitual dietary** | | | |
|---|---|---|---|
| **intake,^{b}** mean (SD) | | | |
| Energy (kcal/d) | 1033 (368) | 1066 (329) | 1024 (369) |
| Protein (g/d) | 32.9 (11.0) | 33.5 (13.6) | 34.3 (13.5) |
| Carbohydrate (g/d) | 146 (49) | 156 (58) | 155 (58) |
| Fat (g/d) | 30.9 (11.4) | 31.8 (13.7) | 32.2 (13.2) |
| Fiber (g/d) | 4.6 (2.8) | 4.9 (3.7) | 4.5 (3.1) |
| Calcium (mg/d) | 228 (94) | 228 (105) | 235 (95) |
| Iron (mg/d) | 6.1 (2.4) | 6.2 (2.7) | 6.1 (2.7) |
| Zinc (mg/d) | 4.4 (1.6) | 4.4 (2.0) | 4.4 (2.0) |

| | | | |
|---|---|---|---|
| Note: Data are number, number (%), mean (SD, median (interquartile range). LC indicates low calcium; RC, regular calcium; ARI, acute respiratory infections; hs-CRP, serum high-sensitivity C-reactive protein; AGP, alpha 1-acid glycoprotein. ^{a}Student's t test, significantly different between RC *us* RC + casei (*P*<.05). ^{b}Assessed using a semi-quantitative food frequency questionnaire. | | | |

**Table 3. Effect of L. casei and calcium on the number of episodes and severity of acute diarrhea assessed by the modified Vesikari score**

| **Variable** | | | **LC** | **RC** | **RC + Casei** |
|---|---|---|---|---|---|
| **All children, n** | | | **124** | **126** | **120** |
| | Mean incidence/child/year | | 0.91 | 0.86 | 1.05 |
| | Mean number of episode (SD) | | 0.40 (0.81) | 0.38 (0.78) | 0.47 (0.87) |
| | | Adjusted RR (95% CI) | 1.00 (ref) | 0.99 (0.62-1.58) | |
| | | | | 1.00 (ref) | 1.21 (0.76-1.92) |
| **Diarrheal children, n^{a}** | | | **33** | **31** | **36** |
| | Mean number of episodes (SD) | | 1.52 (0.87) | 1.55 (0.81) | 1.53 (0.91) |
| | | Adjusted RR (95% CI) | 1.00 (ref) | 1.08 (0.72-1.62) | |
| | | | | 1.00 (ref) | 0.94 (0.63-1.42) |
| | Mean cumulative duration in | | | | |
| | days (SD) | | 4.64 (5.58) | 4.55 (5.06) | 3.62 (4.69) |
| | | Adjusted RR (95% CI) | 1.00 (ref) | 1.18 (0.72-1.92) | |
| | | | | 1.00 (ref) | 0.64 (0.39-1.03) |
| | Mean Vesikari score (SD) | | 3.86 (1.37) | 3.85 (1.09) | 3.96 (1.58) |
| | | Adjusted RR (95% CI) | 1.00 (ref) | 1.02 (0.79-1.32) | |
| | | | | 1.00 (ref) | 1.01 (0.78-1.31) |

| | | | | | |
|---|---|---|---|---|---|
| Note: LC indicates low calcium; RC, regular calcium Data are mean (SD) and Relative Risk (95% Cl); Cl, confidence interval; ref, reference group of comparison. Adjusted for area of living, sex, age, diarrhea and ARI 2 weeks prior to the study, household expenditure and weight for height z score. ^{a}These are all diarrheal children regardless whether fecal samples could be collected. Also, some children had multiple episodes. | | | | | |

**Table 4. Prevalence of rotavirus-related diarrhea and severity of diarrheal disease as assessed by the modified Vesikari score among the groups**

| | **Variable** | **LC** | **RC** | **RC + Casei** |
|---|---|---|---|---|
| Diarrheal children (episode), n | | 33 (50) | 31 (48) | 36 (54) |
| Children (episode) analyzed for | | 24 (29) | 22 (32) | 26 (36) |
| rotavirus, n | | | | |
| **Rotavirus prevalence,** n (%) | | | | |
| | Rotavirus-positive | 9 (31%) | 9 (28%) | 12 (33%) |
| | Rotavirus-negative | 20 (69%) | 23 (72%) | 24 (67%) |
| **Duration of diarrheal episodes** | | | | |
| **(days),** mean (SD) | | | | |
| | Rotavirus-positive | 5.4 (6.0)^{a} | 2.4 (1.7) | 2.3 (1.9) |
| | Rotavirus-negative | 4.1 (5.2) | 4.0 (4.2) | 2.1 (2.1)^{a} |
| **Severity of diarrheal episodes** | | | | |
| **(score),^{b}** mean (SD) | | | | |
| | Rotavirus-positive | 4.7 (1.9) | 4.1 (1.5) | 3.8 (0.7) |
| | Rotavirus-negative | 3.9 (1.6) | 4.5 (1.7) | 4.4 (1.9) |

| | | | | |
|---|---|---|---|---|
| Note: LC indicates low calcium; RC, regular calcium. ^{a}Negative binomial model, significantly different between: LC us RC (relative risk [RR] 2.48; 95% CI: 1.26-4.90; *P* = .01); RC + casei us RC (RR 0.52; 95% CI: 0.28-0.96; *P* = .04); Adjusted for area of living, sex, and age. ^{b}Total Vesikari score of diarrhea (score range 0-18 point; score<11 is mild; score ≥ 11 is severe). Mean values per child were used for children who experienced multiple episodes | | | | |

## Claims

1. A combination of viable *Lactobacillus casei* CRL 431 and calcium for use in a method for reducing the duration of an episode of diarrhea in an individual, wherein said method comprises administering said *L. casei* CRL 431 in an amount of at least 10⁶ CFU per 24 hours and said calcium in an amount of at least 300 mg per 24 hours.

2. A combination for use according to claim 1, wherein the amount of administered calcium is at least 350 mg, preferably at least 400 mg, per 24 hours.

3. A combination for use according to claim 1 or 2, wherein the amount of administered viable *L. casei* CRL 431 is at least 10⁷, preferably at least 10⁸ CFU, per 24 hours.

4. A combination for use according to any one of claims 1-3, wherein said diarrhea is acute diarrhea.

5. A combination for use according to any one of claims 1-4, wherein said individual is an infant up to 6 years of age.

6. A liquid nutritional composition comprising viable *Lactobacillus casei* CRL 431 in an amount of at least 10⁴ CFU per ml and calcium in an amount of at least 0.6 mg per ml, preferably between 10⁵ and 10⁷ CFU per ml of viable *L*. *casei* CRL 431 and between 0.6 and 4 mg/ml of calcium.

7. Nutritional composition according to claim 6 which is a non-fermented milk product, preferably a flavoured non-fermented milk product, more preferably a chocolate flavoured non-fermented milk product.

8. Nutritional composition according to claim 7, which is an infant formula comprising, in addition to the viable *L. casei* CRL 431 and calcium, a carbohydrate source, a lipid source and a protein source.

9. Nutritional composition according to any one of claims 6-8, wherein said *L. casei* CRL 431 constitutes at least 50% of the total amount of bacteria present in said nutritional composition, preferably wherein said *L. casei* CRL 431 is the sole bacterial agent.

10. Nutritional composition according to any one of claims 6-9, further comprising at least one prebiotic compound.

11. Nutritional composition according to any one of claims 6-10, comprising at most 0.1 gram of lactose per 100 ml.

12. Nutritional composition according to any one of claims 6-11, for use as a medicament.

13. Nutritional composition according to any one of claims 6-12, for use in a method for reducing the duration of an episode of diarrhea in an individual, wherein said method preferably comprises administering said *L. casei* CRL 431 in an amount of at least 10⁶ CFU per 24 hours and said calcium in an amount of at least 300 mg per 24 hours.

14. A dry powder comprising calcium and viable *Lactobacillus casei* CRL 431, which upon reconstitution with a suitable liquid, preferably water, provides a nutritional composition according to any one of claims 6-11.

15. Kit of parts comprising:
- a first container comprising a liquid nutritional composition comprising calcium in an amount of at least 0.6 mg per ml, preferably between 0.6 and 4 mg/ml of calcium, and
- a second container comprising viable *Lactobacillus casei* CRL 431 in an amount of at least 10⁶ CFU.
